# EUROPÄISCHE PATENTSCHRIFT

(11) **EP 0 705 582 B1**
(45) Veröffentlichungstag und Bekanntmachung des Hinweises auf die Patenterteilung: **02.11.2000**
(21) Anmeldenummer: 94115801.6
(22) Anmeldetag: 07.10.1994
(51) Int. Cl.: A61F 5/01

(54) **Kniegelenkorthese**
Knee joint orthesis
Orthèse de l'articulation du genou

(43) Veröffentlichungstag der Anmeldung: 10.04.1996
(73) Patentinhaber: Wetz, Hans Henning, Dr., 8708 Männedorf (CH); Hilaire, Jacob, Dr., 8409 Winterthur (CH)
(72) Erfinder: Wetz, Hans Henning, Dr., 8708 Männedorf (CH); Hilaire, Jacob, Dr., 8409 Winterthur (CH)
(74) Vertreter: Kemény AG Patentanwaltbüro

(56) Entgegenhaltungen:
- EP-A- 0 297 766
- WO-A-88/04542
- WO-A-91/04721
- GB-A- 1 534 434

## Beschreibung

Die vorliegende Erfindung betrifft eine Kniegelenkorthese mit Befestigungsmitteln für den Ober- und Unterschenkel, welche mittels zwei Drehelementen verbunden sind.

Bei Verletzungen insbesondere des Bandapparates des menschlichen Kniegelenks wird üblicherweise für den Heilungsprozess eine Knieführungsorthese verordnet. Diese soll eine möglichst physiologische Bewegungsführung des Kniegelenks übernehmen, wobei möglichst unnötige und unnatürliche Krafteinwirkungen auf das Gelenk resp. den Bandapparat vermieden werden sollten. Die Bewegungsführung soll eine stetige Bewegung des Kniegelenks ermöglichen, da eine Stillegung des Gelenks während des Heilungsprozesses der Bänder unerwünscht ist.

Ein grosses Problem beim Erfüllen dieser Anforderung liegt darin, dass es sich bei der Beugung des Kniegelenks nicht um eine einfache Scharnierbewegung handelt, sondern um einen komplexen Bewegungsablauf mit bezüglich der Ober- und Unterschenkel ständig verändernden Drehachse.

Eine Kniegelenkorthese ist beispielsweise in EP 0 297 766 beschrieben. Dort weist eine Kniegelenkorthese mit Befestigungsmitteln für den Ober- und Unterschenkel zwei identische Drehelemente auf. Diese Drehelemente sind als überschlagenes Vierergelenk aufgebaut und bilden damit Gelenke mit variabler Drehachse. Damit wird die Drehachse bezüglich des Ober- und Unterschenkels beim Beugen der Orthese zwar ständig verschoben, allerdings bleibt die Ausrichtung der Drehachse bezüglich des Ober- resp. Unterschenkels stets dieselbe, es findet also lediglich eine Parallelverschiebung der Achse statt.

Eine besondere Ausführungsform eines solchen Drehelementes mit überschlagenem Vierergelenk ist beispielsweise in EP 0 546 330 beschrieben. Dieses weist zusätzliche Mittel auf, welche verhindern, dass Teile des Drehelementes am Knie scheuern können.

Weiter ist aus der GB-A-1 534 434 eine Kniegelenkorthese bekannt, bei welcher die Befestigungsmittel für Unter- und Oberschenkel über Drehelemente miteinander verbunden sind, wobei das eine Drehelement auf der lateralen Seite einen fixen Drehpunkt aufweist und das andere Drehelement auf der medialen Seite über eine Nut-Zapfen-Verbindung einen frei verschiebbaren Drehpunkt aufweist.

Die Aufgabe der vorliegenden Erfindung bestand nun darin, eine Kniegelenkorthese zu finden, welche dem Bewegungsablauf des natürlichen Kniegelenkes in verbessertem Masse zu folgen vermag und insbesondere eine zusätzliche Krafteinwirkung auf das Gelenk und damit auf den Bandapparat beim Beugen und Strecken möglichst verhindert.

Diese Aufgabe wird erfindungsgemäss entsprechend dem Kennzeichen von Anspruch 1 gelöst.

Erfindungsgemäss ist das fixe Drehelement auf der medialen Knieseite angeordnet, was zu einer nicht parallelen Verschiebung der Drehachse der Orthese führt, wie sie praktisch annähernd gleich auch beim menschlichen Knie auftritt. Diese Lage gilt auch für das einen verschiebbaren Drehpunkt aufweisende Drehelement auf der lateralen Knieseite.

Eine bevorzugte Ausführungsform der Erfindung ergibt sich nach Anspruch 2. Die Verwendung eines Kugelgelenks verhindert zuverlässig ein Verklemmen des Drehpunktes des fixen Drehelementes beim Verschwenken der Orthese.

Eine weitere bevorzugte Ausführungsform ergibt sich nach Anspruch 3. Es hat sich gezeigt, dass insbesondere eine Verschiebung des Drehpunktes der Drehachse auf der lateralen (äusseren) Seite des Kniegelenks von ca. 15 mm in den ersten ca. 20° - 30° von der voll gestreckten Position in eine gebeugte Position zusätzliche, von der Orthese hervorgerufene Kräfte auf das Kniegelenk praktisch vollständig vermeidet. Ab ca. 40° Beugung von der voll gestreckten Position bis zu einer annähernd rechtwinkligen Beugung des Kniegelenks (also ca. bis 90°) tritt nur noch eine kleine, annähernd parallele Wanderung resp. Verschiebung der Drehachse des Kniegelenkes bezüglich der Ober- und Unterschenkelbefestigungmittel auf. Durch die erfindungsgemässe Anordnung der beiden unterschiedlichen Gelenke auf der medialen und lateralen Seite des Kniegelenks kann diese Verschiebung der momentanen Drehachse des Kniegelenks praktisch exakt nachvollzogen werden. Dies führt vorteilhafterweise zu praktisch keiner zusätzliche Krafteinwirkung auf das Kniegelenk resp. den Bandapparat, hervorgerufen durch die Bewegung der Orthese.

Vorzugsweise wird das Drehelement mit sich verschiebendem Drehpunkt entsprechend Anspruch 4 praktisch in Form eines überschlagenen Vierergelenks ausgeführt. Ein solches Gelenk ist sehr einfach und damit auch kostengünstig zu realisieren. Die Positionen für die Gelenkstellen und die Längen der beiden Gelenkstangen lassen sich durch den Fachmann bekannterweise einfach derart bestimmen resp. einstellen, dass sich der Drehpunkt der momentanen Drehachse an diesem Gelenk wie gewünscht verschiebt.

Dies wird insbesondere durch die Ausgestaltung nach Anspruch 5 erleichtert, indem die Länge der beiden Gelenkstangen einstellbar ist. Beispielsweise kann dies durch Gewindestangen erreicht werden.

Weiter wird vorzugsweise nach Anspruch 6 vorgeschlagen, die Gelenkpositionen bezüglich der Ober- und Unterschenkelbefestigungsmittel verstellbar auszuführen. Damit lässt sich eine Orthese optimal an das jeweilige Kniegelenk des Trägers anpassen. Dies kann beispielsweise dadurch erfolgen, dass die Verbindung der Drehelemente mit den Befestigungsmitteln mittels einer in gewissen Grenzen verschiebbaren Verschraubung, beispielsweise mit Gleitnuten oder -löchern, realisiert wird.

Weiter sind gemäss Anspruch 7 vorzugsweise Begrenzungselemente zur Einschränkung des Beugebereiches der Orthese vorgesehen. Damit kann sowohl eine Überbeanspruchung des Kniegelenks wie auch der Orthese durch unzulässige Bewegungen vermieden werden.

Weitere bevorzugte Ausführungsformen der Erfindung ergeben sich aus den Ansprüchen 8 resp. 9. Die Schalenresp. Halbschalenform der Ober- resp. Unterschenkelbefestigungsmittel ergibt eine besonders zuverlässige und doch schonende Fixierung der Orthese am Bein des Orthesenträgers. Solche Formen können entweder individuell aufgrund eines Abdruckes des Beines des Orthesenträgers erstellt werden oder es können vorgeformte, mit anpassbaren Einlagen versehene Standardformen eingesetzt werden. Selbstverständlich sind auch alle anderen bekannten Befestigungsmittel wie beispielsweise Schienen und Bänder verwendbar.

Durch die Verwendung einer erfindungsgemässen Knieorthese wird es möglich, eine bezüglich des Gelenks und des Bandapparates praktisch kräftefreie Führung der Bewegung des Kniegelenks über mindestens den wichtigsten Beugebereich, von voll durchgestreckt bis zu wenigstens rechtwinklig abgebogen, zu ermöglichen. Damit kann eine wesentliche Verbesserung des Heilungsprozesses des Bandapparates gewährleistet werden, ohne dass diesem neue oder zusätzliche Schäden zugeführt werden.

Ein Ausführungsbeispiel der Erfindung wird nachstehend anhand von Zeichnungen noch näher erläutert. Es zeigen
Fig. 1 die schematische Frontansicht einer erfindungsgemässen Orthese;
Fig. 2 die schematische Ansicht eines Gelenkes mit verschiebbarer Achse.

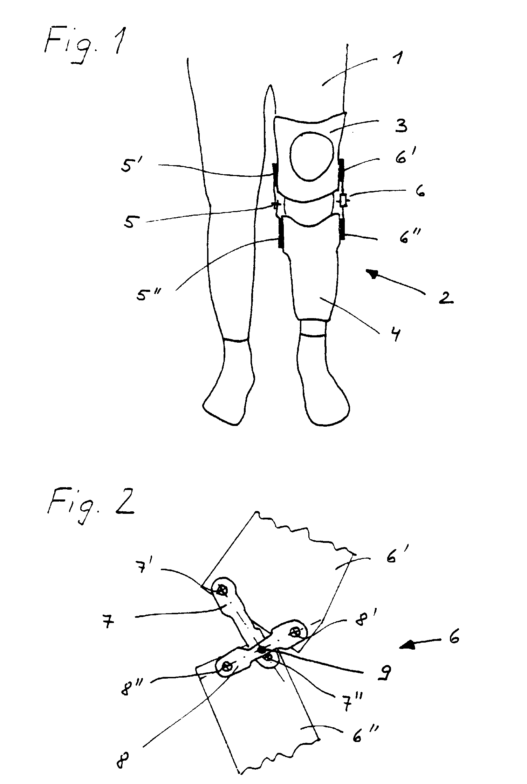

Figur 1 zeigt die Vorderansicht der Beine einer Person, wobei am linken Bein 1 eine erfindungsgemässe Orthese 2 angebracht ist. Die Orthese 2 besteht aus einem Oberschenkelbefestigungsmittel 3 und einem Unterschenkelbefestigungsmittel 4, welche hier beispielsweise in Schalenbauweise ausgeführt sind. Die Befestigungsmittel 3 und 4 sind durch zwei Drehelemente 5 und 6 derart verbunden, dass bei einem Beugen des Beines 1 die Beugebewegung des Knies durch die Orthese 2 genau geführt wird. Dies wird erfindungsgemäss dadurch erreicht, dass das mediale Drehelement 5 als einfaches Gelenk, hier beispielsweise als Kugelgelenk, ausgeführt ist. Das laterale Gelenk 6 ist hingegen als Gelenk mit variabler Drehachse ausgebildet, beispielsweise als überschlagenes Vierergelenk.

Um die Achsen der Drehelemente 5,6 genau dem entsprechenden Kniegelenk anzupassen, werden die Drehelemente 5,6 vorzugsweise mittels einstellbaren Verbindungsstücken 5',5'' resp. 6',6'' mit den Befestigungsmitteln 3,4 verbunden. Beispielsweise kann dies durch Verschraubungen durch Gleitlöcher erfolgen. In Figur 2 wird schematisch beispielshaft der Aufbau des Drehelementes 6 dargestellt. Die beiden Verbindungsstücke 6' und 6'' des Gelenkes 6 sind durch zwei über Kreuz geführte Verbindungsstangen 7 und 8 miteinander verbunden. Jede der beiden Verbindungsstangen 7,8 ist drehbar über Gelenke 7',7'' resp. 8',8'' an den Verbindungsstücken 6' und 6'' befestigt. Das momentane Drehzentrum 9 des Gelenkes befindet sich auf dem Kreuzungspunkt der beiden Verbindungsachsen der Verbindungsstangen 7 und 8. Es ist aus dieser Anordnung ersichtlich, dass bei einer Verdrehung des Drehelementes 6 dieses momentane Drehzentrum 9 wandert. Die Grösse der Wanderung dieses Drehzentrums 9 hängt im wesentlichen von der Position der Gelenke 7',7'',8',8'' und der Länge der Verbindungsstangen 7 und 8 ab, resp. lässt sich durch entsprechende Dimensionierung dieser Grössen einstellen. Vorzugsweise werden diese Grössen derart bemessen, dass die Wanderung des Drehzentrums 9 über einen Drehbereich von ca. 90° (von durchgestrecktem Knie bis rechtwinklig durchgebogenem Knie) etwa 15mm beträgt.

Durch diese Ausführung der beiden Drehelemente 5 und 6 wird eine im wesentlichen der effektiven Bewegung der Drehachse des Kniegelenks entsprechende Verschiebung der momentanen Drehachse der Orthese 2 erreicht. Dies führt vorteilhafterweise dazu, dass unerwünschte Krafteinwirkungen auf das Kniegelenk und den Bandapparat durch die Bewegung der Orthese weitestgehend vermieden werden können. Damit diese Bewegung nicht zu einer Verkantung des einfachen Gelenks 5 führt, ist dieses vorzugsweise als Kugelgelenk ausgeführt.

Es hat sich gezeigt, dass beim Anpassen der Orthese 2 an das Bein 1 die Verbindungsstücke 5',5'' resp. 6',6'' zuerst lose, d.h. leicht verschiebbar, mit den Befestigungsmitteln 3 und 4 verbunden werden sollen. Vorteilhafterweise sind daher diese Verbindungen beispielsweise durch Verwendung von Gleitlöchern verschiebbar, wie vorgängig bereits erwähnt. Anschliessend wird das Knie einmal von der voll durchgestreckten Position in eine annähernd rechtwinklige Position gebeugt und erst danach die Verbindungsstücke 5',5'' rsp. 6',6'' unverrückbar mit den Befestigungsmitteln 3 und 4 verbunden.

Wie durch Messungen festgestellt wurde, konnten durch eine derartige erfindungsgemässe Orthese die auf das Kniegelenk einwirkenden Kräfte praktisch vollständig vermieden werden, im Gegensatz zur symmetrischen Verwendung von zwei gleichen Gelenken, wie es bisher üblich war.

Im weiteren wird vorzugsweise gleichzeitig eine Vereinfachung der Mechanik einer solchen Knieorthese erreicht, indem nur ein einziges, verhältnismässig kompliziert und aufwendig aufgebautes Gelenk mit verschiebbarer Achse verwendet werden muss, während das andere Gelenk als einfaches und damit auch kostengünstiges Gelenk ausgebildet ist.

## Patentansprüche

1. Kniegelenkorthese (2) mit Befestigungsmitteln (3,4) für den Ober- und Unterschenkel, welche mittels zwei einen Drehpunkt (9) aufweisenden Drehelementen (5,6) verbunden sind, wobei das eine Drehelement (5) beim Verschwenken der Orthese (1) einen bezüglich der Befestigungsmittel (3,4) fixen Drehpunkt und das andere Drehelement (6) einen bezüglich der Befestigungsmittel (3,4) sich verschiebenden Drehpunkt (9) aufweist, dadurch gekennzeichnet, dass das fixe Drehelement (5) auf der medialen Knieseite angeordnet ist.

2. Kniegelenkorthese nach Anspruch 1, dadurch gekennzeichnet, dass das fixe Drehelement(5) ein Kugelgelenk ist.

3. Kniegelenkorthese nach Anspruch 1 oder 2, dadurch gekennzeichnet, dass beim Drehelement (6) mit sich verschiebendem Drehpunkt (9) dieser sich beim Verschwenken der Orthese um im wesentlichen 90° um im wesentlichen 15mm verschiebt.

4. Kniegelenkorthese nach einem der Ansprüche 1 bis 3, dadurch gekennzeichnet, dass das Drehelement (6) mit sich verschiebendem Drehpunkt (9) als überschlagenes Vierergelenk ausgebildet ist, wobei die Befestigungsmittel (6',6'') durch zwei sich überkreuzende, gelenkig an den Befestigungsmitteln angeordnete Gelenkstangen (7,8) verbunden sind.

5. Kniegelenkorthese nach Anspruch 4, dadurch gekennzeichnet, dass die Gelenkstangen (7,8) in der Länge einstellbar sind.

6. Kniegelenkorthese nach einem der Ansprüche 1 bis 5, dadurch gekennzeichnet, dass die Lage der Drehelemente (5,6) bezüglich der Ober- resp. Unterschenkelbefestigungsmittel (3,4) einstellbar ist.

7. Kniegelenkorthese nach einem der Ansprüche 1 bis 6, dadurch gekennzeichnet, dass der Verdrehwinkel der Drehelemente (5,6) durch Begrenzungs- resp. Anschlagmittel einschränkbar ist.

8. Kniegelenkorthese nach einem der Ansprüche 1 bis 7, dadurch gekennzeichnet, dass die Befestigungsmittel (3,4) für den Ober- resp. Unterschenkel in Schalenbauweise bestehen.

9. Kniegelenkorthese nach Anspruch 8, dadurch gekennzeichnet, dass die Befestigungsmittel (3,4) in Halbschalenbauweise bestehen.

## Claims

1. A knee-joint orthesis (2) having means (3, 4) for mounting the orthesis to the thigh and to the lower leg, which means are connected by two rotation elements (5, 6) having each point of rotation (9), wherein upon a pivoting of the orthesis one of the rotation elements (5) features a fixed point of rotation relative to the mounting means (3, 4) and the other rotation element (6) features a displaceable point of rotation (9) relative to the mounting means, characterized in that the rotation element which features the fixed point of rotation is located on the medial knee side.

2. The knee-joint orthesis of claim 1, wherein the fixed rotation element (5) is a ball and socket joint.

3. The knee-joint orthesis of claim 1 or 2, wherein the point of rotation of the rotation element (6) having the displaceable point of rotation (9) is moving upon a pivoting of the orthesis by substantially 90° by substantially 15 mm.

4. The knee-joint orthesis of any of claims 1 to 3, wherein the rotation element (6) having a displaceable point of rotation (9) is designed as a cross-over quadruple joint, whereby the mounting means (6', 6'') are connected by means of two crossing link rods (7, 8) which are pivotably mounted to said mounting means.

5. The knee-joint orthesis of claim 4, wherein the link rods (7, 8) are of an adjustable length.

6. The knee-joint orthesis of any of claims 1 to 5, wherein the location of the rotation elements (5, 6) is adjustable relative to means (3, 4) for mounting the orthesis to the thigh and the lower leg respectively.

7. The knee-joint orthesis of any of claims 1 to 6, wherein the angle of pivotement of the rotation elements (5, 6) is limitable by means of limiting or abutment means respectively.

8. The knee-joint orthesis of any of claims 1 to 7, wherein the means (3, 4) for mounting the orthesis to the thigh and to the lower leg are of a shell design.

9. The knee-joint orthesis of claim 8, wherein the mounting means (3, 4) are of a half shell design.

## Revendications

1. Orthèse de l'articulation du genou (2), comprenant des moyens de fixation (3, 4) pour la cuisse et la jambe, ces moyens étant reliées par deux éléments d'articulation (5, 6) présentant chacun un point d'articulation (9), l'un des éléments d'articulation (5) présentant, lors du pivotement de l'orthèse, un point d'articulation fixe par rapport aux moyens de fixation (3, 4) et l'autre élément d'articulation (6) un point d'articulation (9) se déplaçant par rapport aux moyens de fixation (3, 4), caractérisée en ce que l'élément d'articulation fixe (5) est disposé du côté médiale du genou.

2. Orthèse de l'articulation du genou selon la revendication 1, caractérisée en ce que l'élément d'articulation fixe (5) est un joint à bille.

3. Orthèse de l'articulation du genou selon la revendication 1 ou 2, caractérisée en ce que dans l'élément d'articulation (6) à point d'articulation déplaçable (9) ce dernier se déplace lors d'un pivotement de l'orthèse d'essentiellement 90° d'une distance d'essentiellement 15 mm.

4. Orthèse de l'articulation du genou selon l'une des revendications 1 à 3, caractérisée en ce que l'élément d'articulation (6) à point d'articulation déplaçable (9) est un joint quadruple basculant, les moyens de fixation (6', 6'') étant alors reliées par deux barres d'articulation (7, 8) se croisant et montées de manière pivotable aux moyens de fixation.

5. Orthèse de l'articulation du genou selon la revendication 4, caractérisée en ce que la longueur des barres d'articulation (7, 8) est réglable.

6. Orthèse de l'articulation du genou selon l'une des revendications 1 à 5, caractérisée en ce que la position des éléments d'articulation (5, 6) est réglable par rapport aux moyens de fixation (3, 4) pour la cuisse et la jambe.

7. Orthèse de l'articulation de genou selon l'une des revendications 1 à 6, caractérisée en ce que l'angle de pivotement des éléments d'articulation (5, 6) peut être limité par des moyens de limitation ou de butée.

8. Orthèse de l'articulation du genou selon l'une des revendications 1 à 7, caractérisée en ce que les moyens de fixation (3, 4) pour la cuisse et la jambe comprennent une construction en monocoque.

9. Orthèse de l'articulation de genou selon la revendication 8, caractérisée en ce que les moyens de fixation (3, 4) comprennent une construction en semi-monocoque.
